(19) Europäisches Patentamt
European Patent Office
Office européen des brevets

(11) Veröffentlichungsnummer: **0 222 970**
**B1**

(12) **EUROPÄISCHE PATENTSCHRIFT**

(45) Veröffentlichungstag der Patentschrift: **24.10.90**

(21) Anmeldenummer: **86109365.6**

(22) Anmeldetag: **09.07.86**

(51) Int. Cl.$^5$: **C 07 D 277/64,** C 07 D 277/70, C 07 D 263/56, C 07 D 293/12, C 07 D 215/10, C 07 D 213/04

# (54) Sulfoalkylierungsverfahren.

(30) Priorität: **21.11.85 DE 3541098**

(43) Veröffentlichungstag der Anmeldung: **27.05.87 Patentblatt 87/22**

(45) Bekanntmachung des Hinweises auf die Patenterteilung: **24.10.90 Patentblatt 90/43**

(84) Benannte Vertragsstaaten: **BE CH DE GB LI**

(56) Entgegenhaltungen:
**EP-A-0 064 667**
**DE-A-2 165 167**
**DE-A-2 238 271**
**DE-A-2 803 493**
**DE-C-2 825 246**
**FR-A-2 188 605**

**CHEMICAL ABSTRACTS, Band 91, Nr. 10, 3. September 1979, Columbus, Ohio, USA; ANON "Preparation of sulfoalkyl quaternary salts" Seite 59, Spalte 1, Zusammenfassung Nr. 75681k**

(73) Patentinhaber: **Agfa-Gevaert AG**
**D-5090 Leverkusen 1 (DE)**

(72) Erfinder: **Hünicke, Wolfgang, Dipl.-Ing.**
**Cohnenhofstrasse 29**
**D-5000 Köln 71 (DE)**
Erfinder: **Gauglitz, Reinhard**
**Fichtestrasse 27**
**D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 222 970 B1

Courier Press, Leamington Spa, England.

**Beschreibung**

Die Erfindung betrifft ein Sulfoalkylierungsverfahren, d.h. ein Verfahren bei dem organische alkylierbare Verbindungen, insbesondere Amine durch Umsetzung mit geeigneten Reagentien sulfoalkyliert werden. Die Erfindung ist besonders gerichtet auf ein Verfahren zur Sulfoalkylierung tertiärer Amine, wobei die betreffenden Sulfoalkylquartärsalze erhalten werden.

Die Einführung von Sulfoalkylgruppen erweist sich in der Regel immer dann von Vorteil, wenn es erforderlich ist organischen Verbindungen hydrophile bzw. anionische Eigenschaften zu verleihen, z.B. bei der Synthese von Farbstoffen, Textilhilfsmitteln, Arznei- und Pflanzenschutzmitteln. Wenn Aminoverbindungen sulfoalkyliert werden, dann erhält man in der Regel amphotere Verbindungen, d.h. Verbindungen, die sowohl saure als auch basische Gruppen enthalten. Von besonderer Bedeutung sind sulfoalkylgruppenhaltige Quartärsalze, die durch Sulfoalkylierung tertiärer Amine erhalten werden können und die in sich durch kovalente Bindungen miteinander verbunden sowohl positiv geladene als auch negativ geladene Gruppen aufweisen. Verbindungen des letztgenannten Typs werden auch als Betaine bezeichnet. Sie spielen in einer Reihe von technischen Prozessen eine wichtige Rolle. Dabei werden diese Betaine entweder als solche direkt verwendet, z.B. in der Galvanotechnik, oder als Zwischenprodukte weiteren Umsetzungen unterworfen. Zur Verwendung der Sulfoalkylbetaine als Zwischenprodukte ist es technisch vorteilhaft, diese zunächst zu isolieren, de hiberbei eine höhere Produktreinheit erzielt werden kann, die sich günstig auf die Qualität der Folgeprodukte auswirkt und zudem rationelle Produktionsverfahren ermöglicht.

Als Zwischenprodukte mit erforderlicher hoher Reinheit spielen die Sulfoalkylbetaine beispielsweise eine bedeutende Rolle bei der Synthese der als spektrale Sensibilisierungsfarbstoffe für lichtempfindliche Materialien, insbesondere fotografische Silberhalogenidemulsionen verwendeten Polymethinfarbstoffe.

Die einfachste und zur Zeit immer noch am meisten angewandte Methode zur Einführung von Sulfoalkylgruppen ist die Umsetzung von Aminen mit Sultonen, beispielsweise nach Helberger, Liebigs Ann. Chem. *565* (1949), 2. Die Anwendung dieser Umsetzung bie der Herstellung von spektralen Sensibilisierungsfarbstoffen ist beispielsweise in DE—C—929 080 beschrieben. Ein großer Nachteil dieser Reaktion ist, daß insbesondere die niederen Sultone, wie 1,3-Propansulton, eine starke mutagene und cancerogene Wirkung zeigen [Druckrey et al, Z. Krebsforsch, *75* (1970), 69].

Es hat daher nicht an Versuchen gefehlt, die bei der Sulfoalkylierung von den Sultonen ausgehende Gefährdung zu vermindern oder gar völlig auszuschalten. Eine Möglichkeit zur Verringerung des von Sultonen ausgehenden Gesundheitsrisikos wird beispielsweise darin gesehen, daß das Sulton durch weniger gesundheitsgefährdende Sulfoalkylierungsmittel ersetzt wird, wobei solche Ersatzstoffe nach Möglichkeit vergleichbar reaktiv wie das Sulton sein sollen. Derartige Verfahren sind beispielsweise beschrieben in DE—A—28 03 493, DE—A—28 25 246, DD—A—139 577, DE—A—29 09 200, EP—A—0 034 279, EP—A—0 055 428, EP—A—0 064 667. Bei den betreffenden normalerweise sulton freinen Sulfoalkylierungsmitteln handelt es sich beispielsweise um Hydroxyalkansulfonsäuren (in wäßriger Lösung), O-Sulfoalkylisoharnstoffverbindungen, Bissulfoalkylether oder Arylsulfoalkylether. Obwohol die genannten Sulfoalkylierungsmittel sich in vielen Fällen zumindest bei kleinen Ansätzen als Ersatzstoff für Sultone eigenen, haftet ihnen doch der grundsätzliche Nachteil an, daß die betreffenden Reaktionen in stark acidem Medium ablaufen, so daß sich ihr Einsatz bei der Sulfoalkylierung säureempfindlicher sulfoalkylierbarer Verbindungen verbietet. Eine weiterer Nachteil, insbesondere beim Einsatz der zuletzt genannten Sulfoalkylierungsmittel, besteht darin, daß sie entweder, wenn sie ohne Lösungsmittel verwendet werden, zu nicht rührbaren und schwer aufarbeitbaren Schmelzen führen, oder bei Verwendung von Lösungsmitteln zur Bildung von Schmieren und schlecht handhabbaren flüssigen Phasen Anlaß geben, Umstände, die die Aufarbeitung erschweren, so daß die betreffenden Umsetzungen mit vertretbarem Aufwand nicht in größerem ("technischen") Maßstab durchgeführt werden können.

Bei der Sulfoalkylierung mit Hydroxyalkansulfonsäuren werden diese zusammen mit der sulfoalkylierbaren Verbindung (Amin) auf Reaktionstemperaturen im Bereich von 140 bis 200°C erhitzt, wobei das vorhandene Lösungswasser und das bei der Reaktion gebildete Wasser abdestilliert werden soll.

Das angegebene Verfahren zeigt jedoch erhebliche sicherheits- und verfahrenstechnische Nachteile sowie qualitative Mängel. Zum einen bereitet die Herstellung wasserarmer Hydroxyalkansulfonsäuren sicherheitstechnische Schwierigkeiten. So werden beispielsweise bei der Aufkonzentration wäßriger Hydroxypropansulfonsäure leicht erhebliche Anteile an Propansulton gebildet. Es gelingt nur unter extrem schonenden Bedingungen (Temperatur $< 60°C$, Arbeiten unter Hochvakuum) die Hydroxypropansulfonsäure bis auf einen Restwassergehalt von 2 bis 5% zu entwässern, ohne daß Propansulton gebildet wird. Sicherheit hinsichtlich der Sultonfreiheit besteht jedoch in der betrieblichen Praxis nicht, so daß bei der Handhabung der Verbindung die gleichen Schutzmaßnahmen wie bei den entsprechenden Sultonen erforderlich sind. Aufgrund der z.Z. geltenden Gesetze (Verordnung über gefährliche Arbeitsstoffe vom 11.02.1982) muß sogar Hydroxypropansulfonsäure bereits bei einem Propansultongehalt von = 0,01% als krebserzeugender Arbeitsstoff eingestuft werden. Beim Erhitzen von 95 bis 98%iger Hydroxypropansulfonsäure auf Temperaturen über 140°C zeigt sich, daß bereits nach kurzer Zeit ca. 20% Propansulton gebildet werden, auch wenn noch kein Wasser aus dem System entfernt wird. Daraus ist ersichtlich, daß bei der Quarternierungsreaktion mit Hydroxypropansulfonsäure erhebliche Mengen an Propansulton gebildet werden können. Wird das bei der Cyclisierung zum Propansulton gebildete Wasser aus dem

Reaktionsgemisch entfernt, kann der Anteil an Propansulton noch gesteigert werden, wobei jedoch gleichzeitig, insbesondere bei höheren Konzentrationen und höheren Temperaturen, deutliche Zersetzungserscheinungen im gebildeten Propansulton zu beobachten sind. Bei sicherheitstechnischen Untersuchungen (Differentialthermoanalyse) zeigte sich, daß selbst in reinem Propansulton bereits nach 30 Minuten Standzeit bei 180°C erste Zersetzungerscheinungen auftreten. Bei einem Reaktionsgemisch, das zu ca. 50% aus Propansulton und zu 50% aus Hydroxypropansulfonsäure besteht, werden diese Zersetzungserscheinungen schon bei 170°C nach 30 Minuten Standzeit beobachtet. Die gleichen Zersetzungseigen-schaften werden bei einem Reaktionsgemisch beobachtet, das nur zu 20% aus Propan-sulton und zu 80% aus Hydroxypropansulfonsäure besteht. Wird die Reaktionstemperatur jeweils nur um 10°C gesteigert, so steight die Zersetzungsenergie auf Werte über 30 Watt/kg (autokatalytische Zersetzung), wodurch die Gefahr einer Wärmeexplosion gegeben ist. Ansatzgrößen, die über den Labormaßstab hinausgehen, lassen sich in diesem Fall sicherheitstechnisch nicht mehr beherrschen.

Ein anderer Weg zur Verringerung des von Sulton ausgehenden Gesundheitsrisikos besteht darin, die Anwendung von Sulton nicht grundsätzlich zu vermeiden, sondern so zu gestalten, daß das Sulton möglichst nur innerhalb geschlossener Apparaturen in Einscheinung tritt, so daß eine Kontaminierung der Umwelt und des Personals weitgehend ausgeschlossen ist. Zu diesem Zweck kann das Sulton beispielsweise durch thermische Spaltung unter Ringschluß aus geeigneten sultonfreien Sulfoalkylierungsmitteln gebildet werden. Ein solches Verfahren ist beispielsweise in DE—A—32 25 887 beschrieben. Sulton wird durch thermische Spaltung von Arylsulfoalkylethern erhalten, wobei das gleichzeitig gebildete Phenol destillativ abgetrennt werden kann. Als Vorteil gegenüber den bekannten Verfahren wird die Abwesenheit von Wasser, Alkoholen und Mineralsäuren bei der nachfolgenden Umsetzung mit heterocyclischen Basen angegeben. Das Problem der Solvolyse bei bestimmten heterocyclischen Stickstoffbasen z.B. bei Benzoxazolen wurde hier zwar folgerichtig erkannt, das angegebene Verfahren ist jedoch mit zahlreichen verfahrenstechnischen Problemen und sicherheitstechnischen Risiken behaftet, die eine gewerbliche Nutzung des Verfahrens ausschließen. Zum einen muß die Spaltung im Hochvakuum unter Abdestillieren des Phenols erfolgen, wobei im Falle des Propansultons leicht deutliche Anteile an Sulton mitverdampfen. Zum anderen verläuft die Spaltung nur unvollständig, was eine komplizierte Probennahme und Analytik erfordert. Letztlich ist das gebildete Sulton thermisch instabiler als das reine Sulton, wobei durch die erforderlichen hohen Spaltungstemperaturen z.B. bei 1,3-Propansulton die Gefahr einer Wärmeexplosion und damit des Entweichens von Sulton in die Umgebung greifbar nahe ist.

Auch bei der Herstellung von Sulton durch Wasserabspaltung aus Hydroxyalkansulfonsäuren beispielsweise nach Helberger, Liebigs Ann. Chem. 588 (1954), 78 oder Willems, Bull. Soc. Chem. Belg. 64 (1955), 426 bestehen erhebliche Risiken, die eine gewerbliche Nutzung beeinträchtigen. Bei den für die Entfernung des Wassers und die Destillation des Sultons in Hochvakuum erforderlichen hohen Temperaturen muß stets mit der Gefahr einer Wärmeexplosion gerechnet werden, wenn Störungen des Vakuums oder der Beheizung auftreten. Da lösungsmittelfrei gearbeitet wird, liegt ein sehr energiereiches System vor und die bei Zersetzung des Sultons plötzlich auftretende Reaktionswärme kann nicht schnell genug nach außen abgeführt werden. Bei der nicht immer vermeidbaren Verlängerung der Standzeit auch bei niederen Temperaturen tritt durch autokatalytische Einflüsse der sauren Bestandteile Zersetzung ein. Da die nachfolgende Umsetzung mit Amin in einem zweiten Reaktionsgefäß durchgeführt wird, sind für Analytik (Probenahme, Gehaltsbestimmung) und Durchführung der Reaktion aufwendige Apparaturen erforderlich, wenn eine Gefährdung der Umwelt ausgeschlossen werden soll.

Schließlich werden in DE—A—1 668 655 und DE—A—1 668 656 Verfahren zur Herstellung von 1,3-Propansulton durch Dehydratisierung von 3-Hydroxypropansulfonsäure in Gegenwart von Monochlorbenzol, Toluol und Xylol beschrieben, wobei das abgespaltene Wasser durch azeotrope Destillation entfernt und das reine Propansulton durch Verdampfen des Lösungsmittels und anschließende Destillation des Reaktionsgemisches isoliert wird. Für die Dehydratisierung wird der Temperaturbereich zwischen 110 und 150°C als günstig und der Temperaturbereich zwischen 120 und 135°C als wahrscheinlich optimal angegeben. Der Siedepunkt von Monochlorbenzol liegt bei 132°C. Mit Monochlorbenzol als Lösungsmittel werden bei 130—134°C nach 10 Stunden ca. 85% Propansulton erhalten, während mit Toluol bei 110°C nur ca. 35% un mit Xylol bei 142—145°C unter gleichen Arbeitsbedingungen ca. 50% Propansulton erhalten werden. Bei Verwendungen von Monochlorbenzol sind jedoch bereits nach 4—6 Stunden Dehydratisierungszeit Zersetzungserscheinungen im Reaktionsgemisch zu beobachten; Zersetzungsprodukte scheiden sich in Form einer teerartigen dunklen Masse an dem Rührer und an der Reaktionsbehälterwandung ab und nehmen bei Verlägerung der Reaktionszeit deutlich zu.

Nach 10 Stunden betragen sie bereits ca. 10%, bei Verlängerung über 12—16 Stunden hinaus kann sich die gesamte eingesetzte Hydroxypropansulfonsäure in eine teerartige, in Monochlorbenzol und auch in Wasser unlösliche Masse zersetzen. Bei kürzeren Dehydratisierungszeiten wird zwar die Zersetzung vermieden, jedoch sinkt auch die Propansultonausbeute deutlich ab.

Bei der Verwendung derart hergestellter Propansultonlösungen zur Sulfoalkylierung von tertiären Aminen zeigen sich folgende schwerwiegende Nachteile:

Bei Einsatz von ca. 85%igen Propansultonlösungen mit ca. 10% Zersetzungsprodukt (10 Stunden Reaktionszeit in Monochlorbenzol) werden meist unreine Reaktionsprodukte in schlechter Ausbeute bzw. nicht mehr rührbare Reaktionsprodukte erhalten, die eine Vergrößerung über den Labormaßstab ausschließen.

Bei Einsatz von 50—75%igen Propansultonlösungen mit relativ wenig Zersetzungsprodukt (3—4 Stunden Reaktionszeit in Monochlorbenzol) treten bedingt durch die Anwesenheit von nicht umgesetzter Hydroxyalkansulfonsäure verstärkt Rührprobleme durch Abscheidung von Schmieren auf, die meist zum Abbruch der Versuche führten. Auch wenn die Aufarbeitung der Reaktionsprodukte im Labormaßstab gelingt, werden nur geringe Ausbeute erhalten.

Aufgrund der ungünstigen Sulfoalkylierungsergebnisse durch Einsatz der unreinen Propansultonlösungen scheint es besser, zunächst das Propansulton durch Destillation zu reinigen und konventionell einzusetzen, wobei wieder die anfangs beschriebenen Nachteile des Propansultons (cancerogene und mutagene Eigenschaften) voll zum Tragen kommen.

Auch das in DE—A—1 668 656 beschriebene Verfahren der kontinuierlichen Entfernung der gebildeten Sultonlösung zur Verringerung der Mengen an Zersetzungsprodukten und zur Ausbeutesteigerung ist für Sulfoalkylierungszwecke im technischen Maßstab wenig praktikabel, da

a) eine spezielle, aufwendige und damit erhöht störanfällige Reaktoranlage benötigt wird,

b) eine Probennahme und Analytik bei der gewonnenen Propansultonlösung erforderlich ist (Expositionsrisiko für das Betriebspersonal),

c) ein weiterer Reaktor für die Sulfoalkylierungsreaktion notwendig ist,

d) eine Anreicherung von Zersetzungsprodukten im Propansultonreaktor stattfindet, die ein periodisches Aussetzen und Reinigen des Reaktors erforderlich macht, wobei gegebenenfalls wasserunlösliche Zersetzungsprodukte ökologisch wegen der vermuteten cancerogenen Eigenschaften als sehr bedenklich einzuorden sind.

Die bekannten Verfahren könnten also bestenfalls als aufwendig abzusichernde und zu betreibende Propansultonerzeugungsmethoden angesehen werden; für Sulfoalkylierungszwecke wäre es jedoch einfacher und mit geringerem Risiko behaftet, käufliches 1,3-Propansulton mit den bekannten Gefahren einzusetzen.

Der vorliegenden Erfindung liegt die Aufgabe zugrunde, ein Sulfoalkylierungsverfahren anzugeben, bei dem die geschilderten Nachteile vermieden werden.

Bei dem Verfahren sollen unter betriebstechnischen und ökologischen Gesichtpunkten insbesondere folgende Forderungen berücksichtigt werden:

Verwendung sultonfreier Einsatzstoffe
Vermeidung von Sultonemissionen
Durchführung der Sulfoalkylierungsreaktion in Abwesenheit von Wasser
Hohe Betriebssicherheit bzw. geringe Störungsanfälligkeit
Hohe Qualität (Ausbeute, Reinheit) der Produkte
Ohne Nachteil vergrößerbare Betriebsansätze.

Gegenstand der Erfindung ist ein Verfahren zur Sulfoalkylierung von Aminen durch Umsetzung mit Sultonen, dadurch gekennzeichnet, daß das verwendete Sulton aus der entsprechenden Hydroxyalkansulfonsäure in Gegenwart eines für Sulton inerten Lösungsmittels, in dem das Sulton löslich, die Hydroxyalkansulfonsäure aber praktisch unlöslich ist, durch wasserabspaltende Cyclisierung erzeugt und anschließend mit dem zu sulfoalkylierenden Amin umgesetzt wird.

Es wurde ferner gefunden, daß die Wasserabspaltung aus Hydroxyalkansulfonsäure unter Bildung der entsprechenden Sultone bei Verwendung aromatischer Lösungsmittel, die einen Siedepunkt zwischen 150 und 185°C (unter Normalbedingungen) aufweisen, und Durchführung der Dehydratisierungsreaktion bei Temperaturen bis zur Siedetemperatur des Lösungsmittels so schnell abläuft, daß bereits nach 1 bis 3 Stunden Reaktionszeit Sultongehalte von 98 bis $\geq$ 99% erhalten werden.

Überraschenderweise enthalten dabei die derart hergestellten Sultonlösungen praktisch keine Zersetzungsprodukte, so daß sie in der betreiblichen Praxis mit den gleichen Qualitätsvorteilen wie die aus den reinen Sultonen hergestellten Lösungen verwendet werden können. Der Reaktionsbehälter kann damit bewußt einfach und damit in hohem Maße betriebssicher ausgelegt werden; durch das Entfallen von Probennahmen und Analytik sowie eine praktisch vollständige Umsetzung mit einem zuzugebenden Reaktionspartner wird die Gefährdung des Betriebspersonals und der Umwelt auf ein Minimum reduziert. Die Verwendung aromatischer Lösungsmittel mit einem Siedepunkt zwischen 150 und 185°C unter Normalbedingungen, z.B. mit einem Siedepunkt von 154°C oder höher, und Durchführung des Verfahrens (vorzugweise unter Normalbedingungen) bei Temperaturen bis zur Siedetemperatur des Lösungsmittels ist daher besonders bevorzugt.

Das Verfahren läßt sich in hervorragender Weise auch für die Herstellung (und Verwendung) von 1,4-Butansulton und 2,4-Butansulton aus den entsprechenden Hydroxyalkansulfonsäuren einsetzen, wobei alle vorstehend genannte Vorteile übertragbar sind. Dies ist umso mehr von Bedeutung, da auch die beiden letztgenannten Sultone als cancerogene Arbeiststoffe eingestuft worden sind.

In allgemeiner Form wird das erfindungsgemäße Verfahren wie folgt durchgeführt (allgemeine Vorschrift):

In einen geeigneten Reaktionsgefäß wird wäßrige Hydroxyalkansulfonsäure zusammen mit dem Lösungsmittel, das vorteilhaft in der 1 bis 10 fachen Menge, bezogen auf die wäßrige Hydroxyalkansulfonsäure verwendet sind, zum Sieden erhitzt. Man destilliert zunächst das Lösungswasser und danach das durch die Cyclisierung zum Sulton freigesetzte Reaktionswasser ab. Dabei steigert sich die Siedetemperatur bis zum Siedepunkt des reinen Lösungsmittels. Danach gibt man die bezogen auf die

eingesetzte Menge an Hydroxyalkansulfonsäure äquivalente Menge der zu sulfoalkylierenden Verbindung, z.B. des Amins, zu der Sultonlösung und rührt die gewünschte Reaktionszeit nach. Das gebildete Umsetzungsprodukt wird nach dem Abkühlen gegebenenfalls unter Zufügen eines Hilfslösungsmittels durch Absaugen isoliert. Um das Produkt von anhaftender Mutterlauge zu befreien, wird meist noch mit einem geeigneten Hilfslösungsmittel nachgewaschen. Nach der Trocknung erhält man das Reaktionsprodukt in reiner Form.

Als Lösungsmittel sind besonders aromatische Kohlenwasserstoffverbindungen und deren Derivate mit Siedepunkten (unter Normalbedingungen) zwischen 150°C und 185°C geeignet. Sie müssen jedoch chemisch inert gegenüber den gebildeten Sultonen sein und die Hydroxyalkansulfonsäuren sollen darin praktisch unlöslich sein.

Beispiele

| | |
|---|---|
| Anisol | Kp = 154°C |
| 1-Chlor-2-methyl-benzol | Kp = 158°C |
| Phenetol | Kp = 170°C |
| 1,2-Dichlor-benzol | Kp = 180°C |
| Diethylbenzol | Kp = 181°C |

Lösungsmittel, die funktionelle gegenüber Sulton reaktive Gruppen enthalten wie beispielsweise Phenol und Kresole, kommen nicht infrage. Sehr günstig ist die Verwendung von Lösungsmitteln, in denen das gebildete Quartärsalz schwer löslich ist und in guter Qualität abgeschieden wird. Die Verwendung von Anisol, Phenetol und/oder 1,2-Dichlorbenzol hat sich als sehr vorteilhaft erwiesen.

Durch die Auswahl des Lösungsmittels kann die Reaktionstemperatur für Bildung und Umsetzung des Sultons auf einen gewünschten optimalen Temperaturbereich begrenzt werden. In einigen Fällen ist die aufeinanderfolgende Anwendung verschiedener Lösungsmittel von besonderem Vorteil und zwar, wenn die Cyclisierung zum Sulton und die nachfolgende Quarternierung bei verschiedenen Temperaturen ablaufen sollen. Dabei kann das Lösungsmittel mit dem tieferen Siedepunkt bei der Sultonerzeugung und das Lösungsmittel mit dem höheren Siedepunkt für die Quarternierungsreaktion eingesetzt werden, wobei das erste Lösungsmittel dann abdestilliert wird. Der Anwendungstechnische Vorteil ist dabei, daß die Sultonerzeugung und die Quarternierungsreaktion unter den jeweils optimalen Temperaturbedingungen ablaufen.

Hilfslösungsmittel werden nach Beendigung der Quaternierungsreaktion z.B. zu folgenden Zwecken eingesetzt:

1. um die Quatärsalzabscheidung zu vervollständigen,
2. um das Reaktionsgemisch zu verdünnen,
3. um die Reaktionsnebenprodukte auszuswaschen und
4. um die Trocknung zu erleichtern.

Für die Hilfslösungsmittel gelten dahr nicht die gleichen Auswahlkriterien wie für das Lösungsmittel. Insbesondere kann es sich hierbei sogar als vorteilhaft erweisen, wenn solche Hilfslösungsmittel gegenüber Sulton reaktionsfähig sind, um nach der Quaternierungsreaktion eventuell noch vorhandenes überschüssiges Sulton chemisch zu binden und unschädlich zu machen. Als Hilfslösumgsmittel kommen beispielsweise Alkohole wie z.B. Methanol, Ethanol, n-Propanol, i-Propanol, oder Ketone wie z.B. Aceton zur Anwendung.

Bei den erfindungsgemäß zu sulfoalkylierenden Verbindungen handelt es sich im wesentlichen um Amine, insbesondere tertiäre Amine. Von besonderem Interesse sind hierbei heterocyclische Basen der allgemeinen Formel I

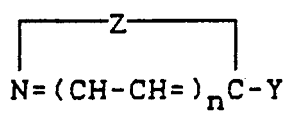

I

worin bedeuten:

Y Wasserstoff, Halogen, gegebenenfalls substituiertes Alkyl oder Alkenyl mit bis zu 6 C-Atomen, Alkoxy, Alkylthio oder Mercapto;

Z den erforderlichen Rest zur Vervollständigung eines 5- oder 6-gliedrigen heterocyclischen Ringes, an den gegebenenfalls weitere Ringe ankondensiert sein können;

n 0 oder 1.

Ein durch Y dargestellter Alkylrest ist beispielsweise Methyl, Ethyl oder ein durch Halogen, Cyan, Alkoxy, Carboxyl oder Alkoxycarbonyl substituierter Alkylrest. Ein durch Y dargestellter Alkoxy- oder Alkylthiorest kann beispielsweise unsubstituiert oder durch Carboxyl, Alkoxycarbonyl oder Sulfo

substituiert sein. Wenn Y eine Mercaptogruppe bedeutet, dann wird die betreffende heterocyclische Base bei dem erfindungsgemäßen Sulfoalkylierungsverfahren in der Regel zweifach sulfoalkyliert, nämlich einmal an dem heterocyclischen Stickstoffatom und einmal an der Mercaptogruppe; letztere wird hierbei in eine Sulfoalkylthiogruppe umgewandelt.

Y kann beispielsweise weiterhin eine Methinkette mit 1, 3 oder 5 Methingruppen bedeuten, an deren Ende sich meist über die 2-Stellung angeknüpft eine N-alkylierte heterocyclische Base befindet, wie sie in der Chemie der Cyaninfarbstoffe bekannt sind. Hierzu sei verwiesen auf F. M. Hamer, "The Cyanine Dyes and Related Compounds", (1964), Interscience Publishers John Wiley and Sons. Verbindungen der Formel I, in der Y in der erwähnten Weise definiert ist, werden als "entquarternierte Cyaninfarbstoffe" bezeichnet. Falls solche entquarternierte Cyaninfarbstoffe nach den Verfahren der Erfindung umgesetzt werden, sind die unmittelbaren Verfahrensprodukte ohne weitere Umsetzung als Sensibilisierungsfarbstoffe geeignet.

Die durch Z vervollständigten heterocyclishen Gruppen bestehen im wesentlichen aus einem 5- oder 6-gliedrigen heterocyclischen Ring; daran können weitere Ringe ankondensiert sein wie Benzol-, Naphthalin- oder auch heterocyclische Ringe, die auch weiter substituiert sein können; infrage kommen die aus der Klasse der Cyaninfarbstoffe bekannten Heterocyclen, wie z.B.; Pyrrolin (z.B. 4,4-Dimethyl-pyrrolin), Oxazolin (z.B. 4,4-Dimethyloxazolin), Thiazolin (z.B. 5-Methylthiazolin), Selenazolin, Indolin (z.B. 3,3-Dimethylindolin, 3,3-Dimethyl-5-methoxyindolin, 3,3-Dimethyl-5-diethylaminoindolin), Imidazol (z.B. 1-Ethyl-5-trifluormethylbenzimidazol, 1-Methyl-5-chlorbenzimidazol, 1-Ethyl-5,6-dichlor-benzimidazol, 1-Ethyl-5-cyanbenzimidazol, 1-Methyl-5-carbethoxybenzimidazol, 1-Ethyl-5-acetylbenzimidazol, 1-Methylbenzimidazol-5-sulfonsäurepyrrolidid, 1-Ethyl-benzimidazol-5-sulfonsäuredimethylamid, 1-Ethyl-5-phenylthiobenzimidazol, 1-Methyl-5-methylthiobenzimidazol, 1-Methyl-5-chlor-6-methylthiobenzimidazol), Oxazol (z.B. 4-Methyloxazol, 4,5-Diphenyloxazol, 4-Methyl-5-carbethoxyoxazol, Benzoxazol, 5-Chlorbenzoxazol, 5-Phenylbenzoxazol, 6-Methoxybenzoxazol, 5-Methoxybenzoxazol, 5-Methyl-6-methoxybenzoxazol, 5-Brombenzoxazol, 5-Iodbenzoxazol, Naphtho[2,1-d]oxazol, Naptho[1,2-d]oxazol, Naphtho[2,3-d]-oxazol, 4,5,6,7-Tetrahydrobenzoxazol, Benzofuro[2,3-f]benzoxazol), Thiazol (z.B. 4-Methyl-thiazol, 4-Phenylthiazol, 4-Methyl-thiazol-5-acrylsäureethylester, Benzthiazol, 5-Methylbenzthiazol, 6-Methylbenzthiazol, 5-Chlorbenzthiazol, 5-Methoxybenzthiazol, 6-Methoxybenzthiazol, 5,6-Dimethylbenzthiazol, 5,6-Dimethoxybenzthiazol, 5-Methyl-6-methoxybenzthiazol, 5-Brombenzthiazol, 5-Phenylbenzthiazol, 6-Methylthiobenzthiazol, 6-Dimethylaminobenzthiazol, 5-Chlor-6-methoxybenzthiazol, 5,6-Methylendioxybenzthiazol, 6-β-Cyanethoxybenzthiazol, 5-Carbomethoxybenzthiazol, 5-Nitrobenzthiazol, 5-Phenylthiobenzthiazol, 5-Thienylbenzthiazol, 6-Hydroxybenzthiazol, 4,5,6,7-Tetrahydrobenzthiazol, 4-Oxo-4,5,6,7-tetrahydrobenzthiazol, Naphto-[2,1-d]-thiazol, Naphtho[1,2-d]thiazol, 4,5-Dihydronaphtho-[1,2-d]-thiazol, 5-Methoxynaphtho-[1,2-d]-thiazol, 5,7,8-Trimethoxy-naphtho[1,2-d]-thiazol), Selenazol (z.B. Benzselenazol, 5-Methylbenzselenazol, 5,6-Dimethylbenzselenazol, 5-Methoxybenzselenazol, 5-Methyl-6-methoxybenzselenazol, 5,6-Dimethoxybenzselenazol, 5,6-Methylendioxybenzselenazol, 6-Methylbenzselenazol, Naphtho[1,2-d]selenazol), 1,3,4-Oxadiazol (z.B. 5-Methyl-1,3,4-oxadiazol, 5-Phenyl-1,3,4-oxadiazol), 1,3,4-Thiadiazol (z.B. 5-Methyl-1,3,4-thiadiazol, 2,5-Bis-methylthio-1,3,4-thiadiazol, 5-Benzylthio-1,3,4-thiazol, 2-Mercapto-5-methylthio-1,3,4-thiazol, 5-Carbethoxymethylthio-1,3,4-thiadiazol), Pyridin (z.B. 2-Methylpyridin, 4-Methylpyridin), Pyrimidin (z.B. 2-Methyl-4-methylthiopyrimidin), Chinolin (z.B. 6-Methylchinolin, 6-Methoxychinolin, 8-Chlorchinolin, 6-Fluorchinolin, 5,6-Benzochinolin, 6,7-Benzochinolin), Imidazol-[4,5-b]-chinoxalin.

Als Hydroxyalkansulfonsäuren kommen erfindungsgemäß insbesondere solche zur Anwendung, die sich im Verlauf des Verfahrens zu Sultonen der folgenden allgemeinen Formel II cyclisieren lassen:

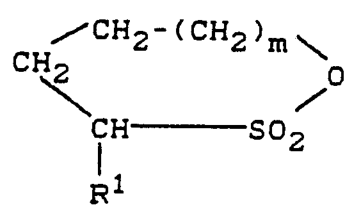

II

worin bedeuten

$R^1$ Wasserstoff oder methyl

m 0 oder 1.

Bevorzugte Beispiele solcher Hydroxyalkansulfonsäuren sind:

3-Hydroxypropansulfonsäure

4-Hydroxypropansulfonsäure

1-Methyl-3-hydroxypropansulfonsäure

Die Herstellung des Sultons durch Wasserabspaltung aus der betreffenden Hydroxyalkansulfonsäure geht rasch und unproblematisch vonstatten. Dabei wird die Bildungsgeschwindigkeit des Sultons durch den Phasenübergang aus der wäßrigen in die Lösungsmittelphase in hervorragender Weise erhöht. Man erhält bereits bei Erreichen der Siedepunkttemperatur des Lösungsmittels (Zeitskala = 0 h) im Reaktionsgefäß Sultongehalte über 80%. Dabei kann das vorhandene Wasser zusammen mit dem

Lösungsmittel abdestilliert werden oder aber über einen Wasserabscheider, verbunden mit einer Lösungsmittelrückführung, entfernt werden. Läßt man nach Erreichen der Siedetemperatur des Lösungsmittels unter nur noch leichtem Sieden weiteres Wasser abdestillieren, so steigt der Sultongehalt bereits nach ca. 1 h auf Werte über 90% an, nach 2 bis 3 h werden Sultongehalte von 95 bis ≧ 99% erzielt, wie beispielsweise aus folgender Tabelle ersichtlich.

Tabelle 1

| Hydroxyalkansulfonsäure | Lösungsmittel | Temperatur/Zeit [°C]/[h] | Sulton | Ausbeute [%] |
|---|---|---|---|---|
| 3-Hydroxypropansulfonsäure | Anisol | 155/0 | 1,3-Propansulton | 87 |
| " | " | 155/1 | " | 97 |
| " | " | 155/2 | " | = 99 |
| 4-Hydroxybutansulfonsäure | Anisol | 155/0 | 1,4-Butansulton | 94 |
| " | " | 155/3 | " | = 98 |
| 1-Methyl-3-hydroxypropan-sulfonsäure | Anisol | 155/0 | 2,4-Butansulton | 84 |
| " | " | 155/2 | " | 95 |
| 1-Methyl-3-hydroxypropan-sulfonsäure | Phenetol | 170/0 | 2,4-Butansulton | 93 |
| " | " | 170/3 | " | 98 |
| 4-Hydroxybutansulfonsäure | 1,2-Dichlor-benzol | 179/0 | 1,4-Butansulton | 88 |
| " | " | 179/1 | " | 99 |
| 4-Hydroxybutansulfonsäure | Diethylbenzol | 180/0 | 1,4-Butansulton | 87 |
| " | " | 180/1 | " | 97 |

Bevorzugte Endprodukte des erfindungsgemäßen Vefahrens, d.h. nach Umsetzung des intermediär gebildeten Sultons mit einem zu sulfoalkylierenden Amin sind Sulfoalkylquartärsalze (Betaine) der folgeden allgemeinen Formel III

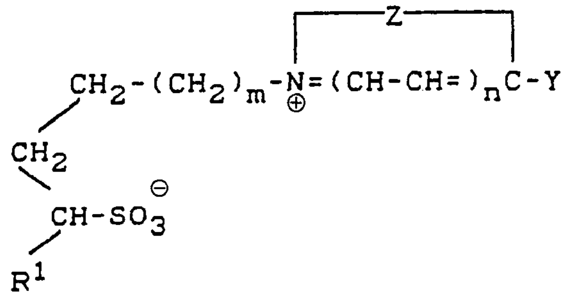

III

worin Y, Z, $R^1$, m und n die bereits angegebene Bedeutung haben.

Die Sulfoalkylquartärsalze fallen bei dem erfindungsgemäßen Verfahren in hoher Ausbeute und in reiner Form an. Weil bei dem erfindungsgemäßen Verfahren nur Lösungen oder allenfalls gut rührbare Kristallsuspensionen vorliegen, eignet es sich auch für die Produktion in größerem (halbtechnischen bis technischen) Maßstab. Das Auftreten von Sulton ist auf die innerhalb geschlossener Apparaturen ablaufenden Reaktionen beschränkt, so daß eine Gefährdung der Umwelt ausgeschlossen ist. Eventuell erforderliche Probenahmen zu Analysezwecken können auf sultonfreie Einsatzstoffe (Hydroxyalkansulfonsäure) beschränkt werden.

Hierbei wird der Gehalt an Hydroxyalkansulfonsäure ermittelt und unter Zugrundelegung dieses Wertes kann die Menge der zu sulfoalkylierenden Verbindung berechnet werden, wobei man im allgemeinen davon ausgehen kann, daß die Cyclisierung zu Sulton quantitativ verläuft.

Falls tatsächlich die Hydroxyalkansulfonsäure, bzw. das daraus erzeugte Sulton in stöchiometrischem Überschuß, bezogen auf die zu sulfoalkylierende Verbindung, eingesetzt wurde, kann dieser Überschuß im Anschluß an die eigentliche Umsetzung z.B. mit reaktiven Hilfslösungsmitteln oder mit Basen zu unschädlichen Produkten zersetzt werden. Da eine praktisch wasserfreie Sultonlösung eingesetzt wird, konnen auch hydrolyseempfindliche Verbindungen nach dem erfindungsgemäßen Verfahren sulfoalkyliert werden.

Das Verfahren weist außerdem eine hohe Betreiebssicherheit bzw. geringe Störanfälligkeit auf. Das Sulton liegt zu keiner Zeit in Substanz, sondern nur in gelöster Form vor. Sofern unter diesen Bedingungen überhaupt eine Zersetzung auftritt, kann die betreffende Reaktionswärme leicht durch Absieden des Lösungsmittels nach außen abgeführt werden, so daß eine unkontrollierte Aufheizung mit der Gefahr einer Wärmeexplosion wirksam vermieden werden kann. Hierbei scheint das Sulton in der Lösung gleichsam in geschützter Form vorzuliegen, wobei möglicherweise der katalytische Einfluß saurer Bestandteile bzw. eventueller Zersetzungsprodukte weitgehend ausgeschahltet ist. Diese wird deutlich durch eine bemerkenswerte Erhöhung der Zersetzungstemperatur des Sultons, Z.B. zeigt eine Lösung von 1,3-Propansulton in Anisol erst oberhalb von 210°C erste Zersetzungserscheinungen (Meßmethode: Differentialthermoanalyse; reines 1,3-Propansulton (ohne Anisol) zersetzt sich bereits bei Temperaturen unterhalb von 180°C). Weitere Vorteile sind schließlich die hohe Qualität der Umsetzungsprodukte hinsichtlich Ausbeute und Reinheit sowie der Umstand, daß die Überstragung (Vergrößerung) des Verfahrens vom Labormaßstab auf den betrieblichen Produktionsmaßstab keine Schwierigkeiten bereitet.

Anhand der folgenden Beispiele soll das erfindungsgemäße Verfahren verdeutlicht werden.

Beispiel 1

Anhydro-2-methyl-3-(3-sulfopropyl)-benzthiazoliumhydroxid

In einen Reaktionsgefäß wurden 175 g Hydroxypropansulfonsäure 80%ig zusammen mit 350 ml Anisol vorgelegt und unter Rühren auf ca. 155°C erhitzt.

Bei Erreichen einer Innentemperatur von ca. 100 bis 105°C begann ein Azeotrop aus Wasser und Anisol abzudestillieren, das nach Kondensation in einem Rückflußkühler ion einen Wasserabscheider geleitet wurde. Während das abgeschiedene Wasser abgelassen wurde, floß das kondensierte Anisol in das Reaktionsgefäß zurück.

Zunächst wurden ca. 50 ml Wasser abdestilliert, wobei der Siedepunkt bis auf 155°C anstieg. Man ließ unter leichtem Sieden noch 1 h nachrühren und trennte dabei über das Ablaßventil des Wasserabscheiders ca. 30 bis 50 ml Anisol und die geringfügigen Restwassermengen ab.

Danach fügte man über ein geeignetes Zulaufgefäß 149 g 2-Methyl-benzthiazol zu und ließ 3 h bei 155°C nachrühren. Während der Nachrührzeit wurde das Reaktionsgemisch durch Abdestillieren von ca. 200 ml Anisol eingeengt. Bereits nach kurzer Zeit schied sich das gewünschte Reaktionsprodukt in Form einer gut rührbaren Kristallsuspension ab.

Nach Beendigung der Reaktionszeit wurde auf ca. 80 bis 90°C abgekühlt und dann wurden 400 ml n-Propanol zur Vervollständigung der Kristallisation zugegeben. Nach dem Abkühlen auf Raumtemperatur

wurde die gut rührbare Kristallsuspension abgesaugt, mit etwas Lösungsmittel nachgewaschen und getrocknet.

Ausbeute: 208 g = 76% der Theorie
Fp: 285°C

Beispiel 2

Anhydro-1-(3-sulfopropyl)-2-methyl-chinolinium-hydroxid

Analog Beispiel 1 wurden 175 g Hydroxypropansulfonsäure 80%ig und 350 ml Anisol auf 155°C erhitzt und ca. 50 ml Wasser sowie 40 ml Anisol abdestilliert.

Man ließ 143 g 2-Methylchinolin gelöst in 100 ml Anisol zutropfen und rührte 3 h bei 155°C nach, wobei noch ca. 200 ml Anisol abdestilliert wurden.

Nach Zugabe von 400 ml Ethanol bei ca. 80°C kühlte man ab und saugte das Quartärsalz ab.

Ausbeute: 153 g = 58% der Theorie
Fp: 275°C

Beispiel 3

Anhydro-1-(3-sulfopropyl)-pyridinium-hydroxid

Analog Beispiel 1 wurden 175 g Hydroxypropansulfonsäure 80%ig und 350 ml Anisol auf 155°C erhitzt und ca. 50 ml Wasser sowie 30 ml Anisol abdestilliert.

Man ließ 79 g Pyridin zutropfen und rührte 3 h bei 155°C nach, wobei noch ca. 100 ml Anisol abdestilliert wurden.

Nach Zugabe von 300 ml n-Propanol bei ca. 90°C kühlte man ab und saugte das Quartärsalz ab.

Ausbeute: 145 g = 72% der Theorie
Fp: 274°C

Beispiel 4

Anhydro-2-methyl-3-(3-sulfopropyl)-5,6-dimethylbenzelenazolium-hydroxid

Analog Beispiel 1 wurden 175 g Hydroxypropansulfonsäure 80%ig und 350 ml Anisol auf 155°C erhitzt und ca. 50 ml Wasser sowie 30 ml Anisol abdestilliert.

Man ließ 224 g 2,5,6-Trimethyl-benzselenazol gelöst bei ca. 60°C in 100 ml Anisol zutropfen und rührte 3 h bei 155°C nach, wobei nach ca. 200 ml Anisol abdestilliert wurden.

Nach Zugabe von 400 ml Ethanol bei ca. 80°C kühlte man ab und saugte das Quartärsalz ab.

Ausbeute: 230 g = 66% der Theorie
Fp: 281°C

Beispiel 5

Anhydro-2-methyl-3-(3-sulfopropyl)-benzoxazolium-hydroxid

Analog Beispiel 1 wurden 175 g Hydroxypropansulfonsäure 80%ig und 350 ml Anisol auf 155°C erhitzt und ca. 50 ml Wasser sowie 30 ml Anisol abdestilliert.

Man ließ 133 g 2-Methyl-benzoxazol zutropfen und rührte 3 h bei 155°C nach, wobei noch ca. 250 ml Anisol abdestilliert wurden.

Nach Zugabe von 400 ml Propanol bei ca. 100°C kühlte man ab und saugte das Quartärsalz ab.

Ausbeute: 165 g = 65% der Theorie
Fp: 259°C

Beispiel 6

Anhydro-2-methyl-3-(3-sulfopropyl)-5-methoxy-benzselenazolium-hydroxid

Analog Beispiel 1 wurden 1.050 g Hydroxypropansulfonsäure 80%ig und 1.500 ml Anisol auf 155°C erhitzt und ca. 300 ml Wasser sowie 200 ml Anisol abdestilliert.

Man ließ 1.356 g 2-Methyl-5-methoxy-benzselenazol zutropfen und rührte 3 h bei 155°C nach, wobei nach ca. 600 ml Anisol abdestilliert wurden.

Nach Zugabe von 2.400 ml Ethanol bei ca. 90 bis 100°C kühlte man ab und saugte das Quartärsalz ab.

Ausbeute: 1.607 g = 77% der Theorie
Fp: 297°C

Beispiel 7

Anhydro-2-methyl-3-(3-suflopropyl)-5-methyl-6-methoxybenzselenazolium-hydroxid

Analog Beispiel 1 wurden 1.050 g Hydroxypropansulfonsäure 80%ig und 2.100 ml Anisol Auf 155°C erhitzt und ca. 300 ml Wasser sowie 200 ml Anisol abdestilliert.

Man ließ eine Lösung von 1.440 g 2,5-Dimethyl-6-methoxy-benzselenazol in 900 ml Anisol schnell zulaufen und rührte 3 h bei 1500°C nach.

Nach Zugabe von 2,4 l n-Propanol bei einer Temperatur von ca. 100°C und kurzem Verrühren wurde auf Raumtemperatur abgekühlt und abgesaugt. Das Produkt wurde durch Nachwaschen mit ca. 1 l Ethanol von

anhaftender Mutterlauge befreit und danach getrocknet.
Ausbeute: 1.722 g = 78% der Theorie
Fp: ca. 300°C

Beispiel 8

Anhydro-2-methyl-3-(3-sulfopropyl)-5-phenyl-benzoxazolium-hydroxid

In einen Reaktionskessel wurden 10,5 kg Hydroxypropansulfonsäure 80%ig und 50 l Anisol unter Rühren auf 155 bis 156°C Innentemperatur erhitzt. Während des Aufheizens destillieren insgesamt 37 l Anisol und 3,18 l Wasser in die Vorlage ab.

Nach Erreichen einer Innentemperatur von 155°C ließ man 1 h nachrühren.

Danach ließ man eine auf 60 bis 80°C erwärmte Lösung von 12,54 kg 2-Methyl-5-phenyl-benzoxazol in 17 l Anisol zu der im Reaktionskessel befindlichen Propansultonlösung zulaufen und rührte das Reaktionsgemisch noch 3 h bei Siedetemperatur nach. Dabei wurden noch ca. 20 l Anisol abdestilliert. Man kühlte die gut rührbare Quartärsalzsuspension auf 90°C ab und ließ dann 25 l Ethanol zulaufen. Nach dem weiteren Abkühlen auf Raumtemperatur wurde das Quartärsalz durch Absaugen isoliert.
Ausbeute: 15,0 kg = 76% der Theorie
Fp: 294°C

Beispiel 9

Anhydro-2,5,6-trimethyl-3-(3-sulfopropyl)-benzothiazolium-hydroxid

In einem Reaktionskessel wurden 16,8 g Hydroxypropansulfonsäure 50%ig und 60 l Anisol unter Rühren auf 155 bis 156°C Innentemperatur erhitzt. Während des Aufheizens destillierten insgesamt 45 l Anisol und ca. 9,51 l Wasser in die Vorlage ab.

Nach Erreichen einer Innentemperatur von 155°C ließ man 1 h nachrühren.

Danach ließ man eine auf 70 bis 80°C erwärmte Lösung von 10,62 kg 2,5,6-Trimethyl-benzthiazol in 20 l Anisol zu der im Reaktionskessel befindlichen Propansultonlösung zulaufen und rührte das Reaktionsgemisch noch 2 bis 3 h bei Siedetemperatur nach. Dabei wurden noch ca. 20 l Anisol abdestilliert. Man kühlte die gut rührbare Quartärsalzsuspension auf ca. 80 bis 90°C ab und ließ dann 20 l Ethanol zulaufen. Nach dem weiteren Abkühlen auf Raumtemperatur wurde das Quartärsalz abgesaugt und mit etwas Aceton nachgewaschen.
Ausbeute: 12,4 kg = 69% der Theorie
Fp: > 300°C

Beispiel 10

Anhydro-2-methyl-3-(3-sulfopropyl)-5-chlor-benzthiazolium-hydroxid

In einem Reaktionskessel, der mit einem Wasserabscheider versehen war, wurden 16,8 kg Hydroxypropansulfonsäure 50%ig und 15 l Anisol unter Rühren auf 155 bis 156°C Innentemperatur erhitzt. Während des Aufheizens destillierten ca. 9,5 l Wasser in den Wasserabscheider ab.

Nach Erreichen einer Innentemperatur von 155°C ließ man noch ca. 3 l Anisol innerhalb 1 h abdestillieren.

Danach ließ man eine auf 60 bis 80°C erwärmte Lösung von 11,01 kg 2-Methyl-5-chlor-benzthiazol in 15 l Anisol zu der im Reaktionskessel befindlichen Propansultonlösung zulaufen und rührte das Reaktionsgemisch noch 3 h bei Siedetemperatur nach. Dabei wurden noch ca. 15 bis 20 l Anisol abdestilliert. Man kühlte die gut rührbare Suspension auf 80°C ab und ließ dann 25 l Ethanol zulaufen. Nach dem weiteren Abkühlen auf Raumtemperatur wurde das Quartärsalz durch Absaugen isoliert und mit Aceton nachgewaschen.
Ausbeute: 15,18 kg = 83% der Theorie
Fp: 291°C

Beispiel 11

Anhydro-2-methyl-3-(4-sulfobutyl)-benzothiazolium-hydroxid

In einem Reaktionsgefäß wurden 92,4 g 4-Hydroxybutansulfonsäure 50%ig zusammen mit 200 ml 1,2-Dichlorbenzol vorgelegt und unter Rühren auf 179°C erhitzt.

Bei Erreichen einer Innentemperatur von ca. 100 bis 105°C begann ein Azeotrop aus Wasser und 1,2-Dichlorbenzol abzudestillieren, das nach Kondensation in einem Rückflußkühler in einen Wasserabscheider geleitet wurde. Während das abgeschiedene Wasser abgelassen wurde, floß das kondensierte 1,2-Dichlorbenzol in das Reaktioonsgefäß zurück.

Es wurden ca. 50 ml Wasser abdestilliert, wobei der Siedepunkt bis auf 179°C anstieg. Man ließ unter leichtem Sieden nach 1 h nachrühren und destillierte dabei ca. 50 ml 1,2-Dichlorbenzol und geringfügiges Restwasser ab.

Danach gab man über ein geeignetes Zulaufgefäß 44,7 g 2-Methylbenzthiazol zu und ließ 2 h bei 180°C nachrühren. Bereits nach kurzer Zeit schied sich das Reaktionsprodukt in Form einer gut rührbaren Kristallsuspension ab.

Nach Beendigung der Reaktionszeit wurde auf ca. 80 bis 90°C abgekühlt und dann wurden 100 ml n-Propanol zur Vervollständigung der Kristallisation zugegeben. Nach dem Abkühlen auf Raumtemperatur

wurde die Kristallsuspension abgesaugt, mit etwas Lösungsmittel nachgewaschen und getrocknet.

Ausbeute: 60,7 g = 71% der Theorie
Fp: 296°C

Beispiel 12

Anhydro-2-methyl-3-(3-sulfo-3-methyl-propyl)-benzthiazolium-hydroxid

In einem Reaktionsgefäß wurden 400 g 1-Methyl-3-hydroxypropansulfonsäure 35%ig zusammen mit 500 ml Anisol vorgelegt und unter Rühren auf 155°C erhitzt.

Bei Erreichen einer Innentemperatur von ca. 100 bis 105°C begann ein Azeotrop aus Wasser und Anisol abzudestillieren, das nach Kondensation in einem Rückflußkühler in einen Wasserabscheider geleitet wurde. Während das abgeschiedene Wasser abgelassen wurde, floß das kondensierte Anisol in das Reaktionsgefäß zurück.

Es wurden ca. 300 ml Wasser abdestilliert, wobei der Siedepunkt bis 155°C anstieg. Man ließ unter leichtem Sieden noch 3 h nachrühren und trennte dabei über das Ablaßventil des Wasserabscheiders ca. 270 ml Anisol und etwas Restwasser ab.

Danach gab man über ein geeignetes Zulaufgefäß 149 g 2-Methylbenzthiazol zu und ließ 3 h bei 160°C nachrühren.

Nach Beendigung der Reaktionszeit wurde auf ca. 80 bis 90°C abgekühlt und dann wurden 250 ml Ethanol zur Vervollständigung der Kristallisation zugegeben. Nach dem Abkühlen auf Raumtemperatur wurde die gut rührbare Kristallsuspension abgesaugt, mit etwas Lösungsmittel nachgewaschen und getrocknet.

Ausbeute: 221 g = 77,5% der Theorie
Fp: 262°C

Beispiel 13

2-(3-Sulfopropylthio)-5-chlor-benzthiazol

In einem Reaktionsgefäß wurden 1,050 g Hydroxypropansulfonsäure 80%ig zusammen mit 1.200 ml Anisol vorgelegt und unter Rühren auf ca. 155°C erhitzt.

Wie im Beispiel 1 wurde ein Azeotrop aus Wasser und Anisol abdestilliert, das nach Kondesation in einem Rückflußkühler in einem Wasserabscheider geleitet wurde. Während das abgeschiedene Wasser abgelassen wurde, floß das kondensierte Anisol in das Reaktionsgefäß zurück.

Zunächst wurden ca. 300 ml Wasser abdestilliert, wobei der Siedepunkt bis auf 155°C anstieg. Man ließ unter leichtem Sieden noch 1 h nachrühren und trennte dabei über das Ablaßventil das Wasserabscheiders ca. 400 ml Anisol und die geringfügigen Restwassermengen ab.

Danach kühlte man die Propansultonlösung auf ca. 100°C ab, fügte 1.209 g 2-Mercapto-5-chlor-benzthiazol zu und ließ ca. 1 h bei 120—130°C nachreagieren. Nach dem Abkühlen auf ca. 80°C wurden 2.400 ml Methanol zugefügt, wobei das gewünschte Reaktionsprodukt auskristallisierte. Nach dem weiteren Abkühlen auf ca. +5°C wurde abgesaugt und mit etwas Methanol nachgewaschen.

Ausbeute: 1386 g = 71% der Theorie
Fp: 188°C

**Patentansprüche**

1. Verfahren zur Sulfoalkylierung von Aminen durch Umsetzung mit Sultonen, dadurch gekennzeichnet, daß das verwendete Sulton aus der entsprechenden Hydroxyalkansulfonsäure in Gegenwart eines für Sulton inerten aromatischen Lösungsmittels mit einem Siedepunkt zwischen 150 und 185°C (unter Normalbedingungen), in dem das Sulton löslich, die Hydroxyalkansulfonsäure aber praktisch unlöslich ist, durch wasserabspaltende Cyclisierung bei Temperaturen bis zur Siedetemperatur des Lösungsmittels erzeugt und anschließend mit dem zu sulfoalkylierenden Amin umgesetzt wird.

2. Verfahren nach Anspruch 1, dadurch gekennzeichnet, daß ein tertiäres Amin verwendet wird.

3. Verfahren nach Anspruch 1 oder 2, dadurch gekennzeichnet, daß als Amin eine heterocyclische Base der allgemeinen Formel 1 verwendet wird

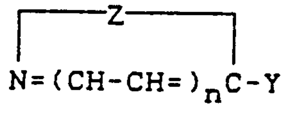

I

worin bedeuten:

Y Wasserstoff, Halogen, gegebenenfalls substituiertes Alkyl oder Alkenyl mit bis zu 6-C-Atomen, Alkoxy, Alkylthio oder Mercapto;

Z den erfordlerlichen Rest zur Vervollständigung eines 5- oder 6-gliedrigen heterocyclischen Ringes, an den gegebenenfalls weitere Ringe ankondensiert sein könen;

n 0 oder 1.

4. Verfahren nach Anspruch 1 oder 2, dadurch gekennzeichnet, daß für die Umsetzung mit dem Amin ein Sulton der folgenden allgemeinen Formel II verwendet wird

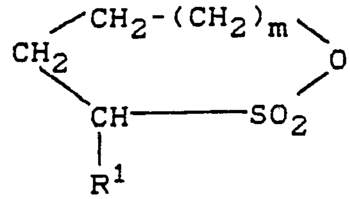

II

worin bedeuten

$R^1$ Wasserstoff oder Methyl

m 0 oder 1.

5. Verfahren zur Herstellung von Sulfoalkylquartärsalzen tertiärer Amine der allgemeinen Formel III

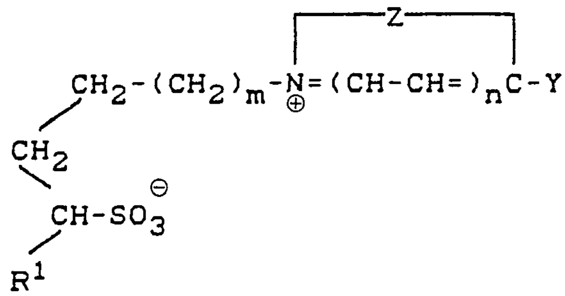

III

worin bedeuten:

Y Wasserstoff, Halogen, gegebenenfalls substituiertes Alkyl oder Alkenyl mit bis zu 6 C-Atomen, Alkoxy oder Alkylthio;

Z den erforderlichen Rest zur Vervollständigung eines 5- oder 6-gliedrigen heterocyclischen Ringes, an den gegebenenfalls weitere Ringe ankondensiert sein können;

m 0 oder 1;

n 0 oder 1,

durch Umsetzung einer heterocyclischen Base der allgemeinen Formel I

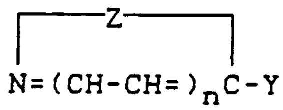

I

worin Y, Z und n die angegebene Bedeutung haben, mit einem Sulton der allgemeinen Formel II

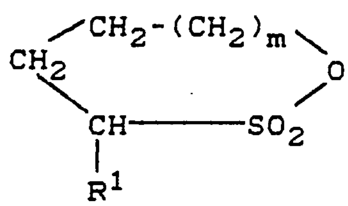

II

worin $R^1$ und m die angegebene Bedeutung haben, dadurch gekennzeichnet, daß das Sulton durch wasserabspaltende Cyclisierung aus der entsprechenden Hydroxyalkansulfonsäure in Gegenwart eines für das Sulton inerten aromatischen Lösungsmittels mit einem Siedepunkt zwischen 150 und 185°C (unter Normalbedingungen), in dem das Sulton löslich, die Hydroxyalkansulfonsäure aber praktisch unlöslich ist, in Form einer Lösung in dem genannten Lösungsmittel bei Temperaturen bis zur Siedetemperatur des Lösungsmittels hergestellt und mit der heterocyclischen Base der allgemeinen Formel I umgesetzt wird.

6. Verfahren zur Herstellung von Sultonen in gelöster Form durch wasserabspaltende Cyclisierung aus Hydroxyalkansulfonsäuren, dadurch gekennzeichnet, daß eine wäßrige Lösung von 3-Hydroxypropansulfonsäure, 4-Hydroxybutansulfonsäure oder 1-Methyl-3-hydroxypropansulfonsäure in Gegenwart von Anisol, Phenetol, 1-Chlor-2-methylbenzol, 1,2-Dichlorbenzol oder Diethylbenzol bis zum Siedepunkt des Lösungsmittels erhitzt wird, bis das Lösungs- und Reaktionswassser abgeschieden ist.

**Revendications**

1. Procédé pour la sulfoalkylation d'amines, par réaction avec des sultones, caractérisé en ce que l'on

obtient la sultone utilisée, à partir de l'acide hydroxyalcanesulfonique correspondant, en présence d'un solvant aromatique inerte pour la sultone, ayant un point d'ébullition entre 150 et 185°C (dans les conditions normales), dans lequel la sultone est soluble mais dans lequel l'acide hydroxyalcanesulfonique est pratiquement insoluble, par cyclisation libérant l'eau, à des températures allant jusqu'à la température d'ébullition du solvant et en ce qu'on la met ensuite à réagir avec l'amine sulfoalkyler.

2. Procédé selon la revendication 1, caractérisé en ce que l'on utilise une amine tertiaire.

3. Procédé selon la revendication 1 ou 2, caracterisé en ce que comme amine, on utilise un base hétérocyclique répondant à la formule générale I

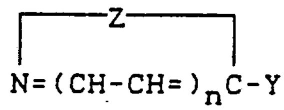

I

dans laquelle:

Y représente un atome d'hydrogène, un atome d'halogène, un groupe alkyle ou un groupe alcényle éventuellement substitué, contenant jusqu'à 6 atomes de carbone, un groupe alcoxy, un groupe alkylthio ou un groupe mercapto;

Z représente le radical requis pour compléter ou noyau hétérocyclique penta- ou hexagonal, auquel peuvent également être accolés d'autres noyaux;

n représente 0 ou 1.

4. Procédé selon la revendication 1 ou 2, caractérisé en ce que, pour la mise en réaction avec l'amine, on utilise une sultone de la formule générale II ci-après:

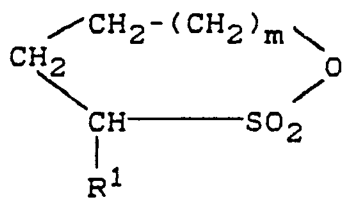

II

dans laquelle:

$R^1$ représente un atome d'hydrogène ou un groupe méthyle

m représente 0 ou 1.

5. Procédé pour la préparation d'un sel quaternaire sulfoalkylé d'amines tertiaires de formule générale III

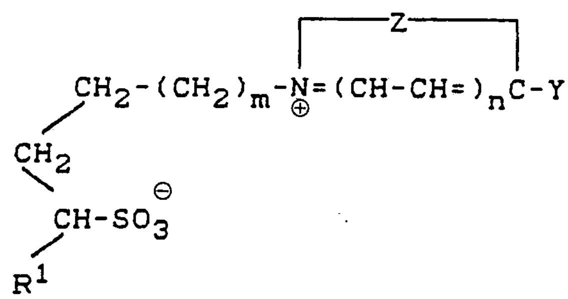

III

dans laquelle:

Y représente un atome d'hydrogène, un atome d'halogène, une groupe alkyle ou un groupe alcényle éventuellement substitué contenant jusqu'à 6 atomes de carbone, un groupe alcoxy ou un groupe alkylthio;

Z représente le radical requis pour compléter un noyau hétérocyclique penta- ou hexagonal, auquel peuvent éventuellement être accolés d'autres noyaux;

m représente 0 ou 1;

n représente 0 ou 1,

par la mise en réaction d'une base hétérocyclique de formule générale I

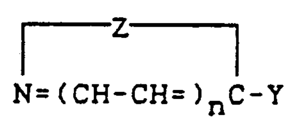

I

dans laquelle Y, Z et n ont la signification indiquée, avec une sultone de formule générale II

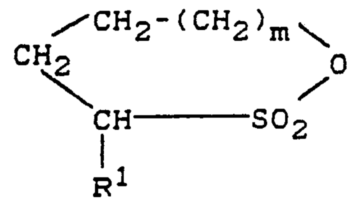

II

dans laquelle $R^1$ et m ont la signification indiquée, caractérisé en ce qu'on prépare la sultone par cyclisation libérant l'eau, à partir de l'acide hydroxyalcanesulfonique correspondant, en présence d'un solvant aromatique inerte pour la sultone, ayant un point d'ébullition entre 150 et 185°C (dans des conditions normales), dans lequel la sultone est soluble mais dans lequel l'acide hydroxyalcanesulfonique est pratiquement insoluble, sous forme d'une solution dans le solvant mentionné, à des températures allant jusqu'à la température d'ébullition du solvant et en ce qu'on la met à réagir avec la base hétérocyclique répondant à la formule générale I.

6. Procédé pour la préparation de sultones sous forme dissoute, par cyclisation libérant l'eau, à partir d'acides hydroxyalcanesulfoniques, caractérisé en ce qu'on chauffe une solution aqueuse de l'acide 3-hydroxypropanesulfonique, de l'acide 4-hydroxybutanesulfonique ou de l'acide 1-méthyl-3-hydroxypropanesulfonique, en présence d'anisol, de phénétol, de 1-chloro-2-méthylbenzène, de 1—2-dichlorobenzène ou de diéthylbenzène, jusqu'au point d'ébullition du solvant, jusqu'à élimination de l'eau de solution et de réaction.

**Claims**

1. A process for the sulphoalkylation of amines by a reaction with sultones, characterised in that the sultone used is produced from the corresponding hydroxyalkane sulphonic acid in the presence of a sultone-inert aromatic solvent having a boiling point from 150°C to 185°C (under normal conditions) in which the sultone is soluble but the hydroxyalkane sulphonic acid is virtually insoluble by cyclisation at temperatures up to the boiling point of the solvent with elimination of water, and the sultone used is then reacted with the amine which is to be sulphoalkylated.

2. Process according to Claim 1, characterised in that a tertiary amine is used.

3. Process according to Claim 1 or Claim 2, characterised in that the amine used is a heterocyclic base corresponding to the general formula I

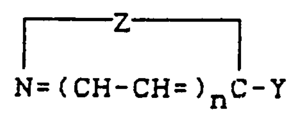

I

wherein

Y denotes hydrogen, halogen, optionally substituted alkyl or alkenyl having up to 6 carbon atoms, alkoxy, alkylthio or mercapto;

Z denotes the group required for completing a 5-membered or 6-membered heterocyclic ring with which additional rings may optionally be condensed;

n denotes 0 or 1.

4. Process according to Claim 1 or Claim 2, characterized in that the sultone used for the reaction with the amine corresponds to the following general formula II

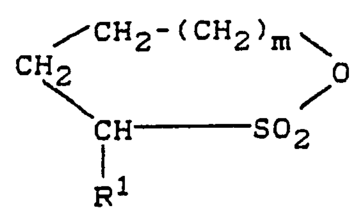

II

wherein

$R^1$ denotes hydrogen or methyl and

m denotes 0 or 1.

5. Process for the preparation of sulphoalkyl quaternary salts of tertiary amines corresponding to the general formula (III)

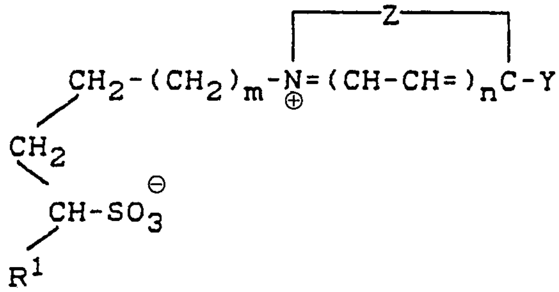

III

wherein

Y denotes hydrogen, halogen, optionally substituted alkyl or alkenyl having up to 6 carbon atoms, alkoxy or alkylthio;

Z denotes the group required for completing a 5-membered or 6-membered heterocyclic ring with which additional rings may optionally be condensed;

m denotes 0 or 1; and

n denotes 0 or 1

by the reaction of a heterocyclic base corresponding to the general formula I

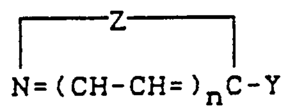

I

wherein Y, Z and n have the meanings indicated above with a sultone corresponding to the general formula II

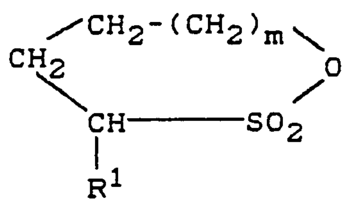

II

wherein $R^1$ and m have the meanings indicated above, characterised in that the sultone is prepared from the corresponding hydroxyalkane sulphonic acid by a cyclisation accompanied by elimination of water in the presence of a sultone-inert aromatic solvent having a boiling point from 150°C to 185°C (under normal conditions) in which the sultone is soluble but the hydroxyalkane sulphonic virtually insoluble, the said sultone being prepared in the form of a solution in the above-mentioned solvent at temperatures up to the boiling point of the solvent and reacted with the heterocyclic base corresponding to the general formula I.

6. Process for the preparation of sultones in a dissolved form from hydroxyalkane sulphonic acids by a water-eliminating cyclisation, characterised in that an aqueous solution of 3-hydroxypropane sulphonic acid, 4-hydroxybutane sulphonic acid or 1-methyl-3-hydroxypropane sulphonic acid is heated to the boiling point of the solvent in the presence of anisole, phenetole, 1-chloro-2-methylbenzene, 1,2-dichlorobenzene or diethylbenzene until the water of solution and water of reaction has been separated off.